# EUROPÄISCHE PATENTSCHRIFT

(11) **EP 1 958 944 B1**
(45) Veröffentlichungstag und Bekanntmachung des Hinweises auf die Patenterteilung: **01.12.2010**
(21) Anmeldenummer: 08101365.8
(22) Anmeldetag: 07.02.2008
(51) Int. Cl.: C07D 307/46

(54) **Verfahren zur Herstellung von 5-Hydroxymethyl-furfural über 5-Acyloxymethyl-furfural als Zwischenprodukt**
Method for producing enantiomer 5-hydroxymethylfurfural with 5-acyloxymethylfurfural as intermediate
Procédé de fabrication de 5-hydroxyméthyle-furfural par 5-acyloxyméthyle-furfural en tant que produit intermédiaire

(30) Priorität: 16.02.2007 DE 102007007629
(43) Veröffentlichungstag der Anmeldung: 20.08.2008
(73) Patentinhaber: Evonik Degussa GmbH, 45128 Essen (DE)
(72) Erfinder: Reichert, Dietmar, 63863, Eschau (DE); Sarich, Martin, 63755, Alzenau (DE); Merz, Friedhelm, 55283, Nierstein (DE)

(56) Entgegenhaltungen:
- EP-A- 1 834 951
- GB-A- 925 812
- CLAUDE MOREAU ET AL: "Recent Catalytic Advances in the Chemistry of Substituted Furans from Carbohydrates and in the Ensuing Polymers" TOPICS IN CATALYSIS, KLUWER ACADEMIC PUBLISHERS-PLENUM PUBLISHERS, NE, Bd. 27, Nr. 1-4, 1. Februar 2004 (2004-02-01), Seiten 11-30, XP019292015 ISSN: 1572-9028

## Beschreibung

Die vorliegende Erfindung bezieht sich auf ein neues Verfahren zur Herstellung von 5-Hydroxymethyl-furfural über ein 5-Acyloxymethyl-furfural als Zwischenprodukt. Die Erfindung betrifft auch ein neues Verfahren zur Herstellung des 5-Acyloxymethyl-furfural-Zwischenprodukts.

5-Hydroxymethyl-furfural mit der folgenden Formel (I) 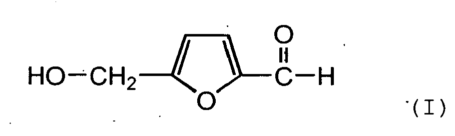 besitzt unter anderem antibakterielle und korrosionsinhibierende Eigenschaften und ist für eine Vielzahl von Reaktionen geeignet. Es ist ohne große Schwierigkeiten möglich, daraus Furfuryldialkohol, -dialdehyd und -dicarbonsäure und deren Derivate herzustellen; desgleichen führt die Hydrierung des Ringes zu difunktionellen 2,5-Tetrahydrofuranderivaten. Auch an C-2 und C-5 unterschiedlich substituierte difunktionelle Furanderivate sind aus 5-Hydroxymethyl-furfural gut zugänglich. Die vorgenannten und andere nützliche aus 5-Hydroxymethyl-furfural herstellbare organische Zwischenprodukte dienen zur Herstellung von zahlreichen chemischen Produkten, wie etwa Lösungsmitteln, oberflächenaktiven Mitteln, Pflanzenschutzmitteln und Harzen. Außerdem wurde über die Verwendung von 5-Hydroxymethyl-furfural zur Behandlung von bösartigen Tumoren berichtet (US-A-5,006,551).

5-Hydroxymethyl-furfural ist ein intramolekulares, dreifaches Dehydratisierungsprodukt von Hexosen (Aldohexosen und Ketohexosen). Nachwachsende Rohstoffe, wie Stärke, Cellulose, Saccharose oder Inulin, sind preiswerte Ausgangssubstanzen zur Herstellung von Hexosen, wie Glucose und Fructose. 5-Hydroxymethyl-furfural ist im Prinzip eine Zwischenstufe der dehydratisierenden Zersetzung von Hexosen zur Lävulinsäure und Ameisensäure, d. h. es kommt entscheidend darauf an, die Reaktion zum richtigen Zeitpunkt abzustoppen. Damit wird die Abtrennung von 5-Hydroxymethyl-furfural von den Ausgangszuckern und Nebenprodukten zu einem wichtigen Schritt bei seiner Herstellung.

Es ist bereits eine große Anzahl verschiedener Verfahren zur Herstellung von 5-Hydroxymethyl-furfural im Labormaßstab bekannt.

Als Katalysator für die Dehydratisierung sind unterschiedliche Säuren bzw. Salze beschrieben worden, z. B. Oxalsäure (vgl. W. N. Haworth et al, J. Chem. Soc. 1944, 667), Salze, wie Pyridinhydrochlorid (vgl. C. Fayet et al, Carbohydr. Res. 122, 59 (1983)), saure Ionenaustauscher (vgl. DE-A-30 33 527) oder Lewis-Säuren, wie Zirkonylchlorid (vgl. SU-A-1 054 349, zit. CA 100, 120866s) oder Bortrifluorid-Etherat (vgl. H. H. Szmant et al, J. Chem. Tech. Biotechnol 31, 135 (1981)).

Für die großtechnische Herstellung von 5-Hydroxymethyl-furfural sollte der eingesetzte Katalysator preiswert und nicht korrosiv sein. Feste, zur Wiederverwendung bestimmte Katalysatoren sind wegen der leichten Bildungsweise von unlöslichen Nebenprodukten deshalb ungeeignet, weil eine Abtrennung des Katalysators (z. B. Ionenaustauscher) von diesen Nebenprodukten unwirtschaftlich bzw. unmöglich ist.

Lewissäuren, wie Zirkonylchlorid oder Aluminiumchlorid, sind aus Korrosionsschutzüberlegungen ebenfalls abzulehnen. Als günstig wird deshalb der Einsatz von Schwefelsäure oder Phosphorsäure angesehen, da in diesem Fall die sauren wässrigen Reaktionslösungen ggf. mit Basen neutralisiert werden können und etwa bei der Verwendung von Calciumhydroxyd oder Calciumcarbonat die Überführung der Katalysatorsäuren in schwer lösliche Salze mit einer Abfiltration möglich ist.

Das Reaktionsmedium der Dehydratisierung von Sacchariden wird durch ihre Löslichkeit bestimmt. Neben Wasser sind insbesondere dipolare, aprotische Lösungsmittel, wie Dimethylformamid oder Dimethylsulfoxid, verwendet worden.

Die iodkatalysierte Umwandlung des Fructoseteiles der Saccharose zu 5-Hydroxymethyl-furfural durch Erhitzen von Saccharose in wasserfreiem Dimethylformamid erfordert neben dem teueren Lösungsmittel außerdem eine aufwendige Aufarbeitung, nämlich Extraktion und Papierchromatographie (vgl. T. G. Bonner et al, J. Chem. Soc. 1960, 787).

In Dimethylsulfoxid werden bei der Zersetzung von Fructose mit unterschiedlichen Katalysatoren gute Ausbeuten (> 90%) an 5-Hydroxymethyl-furfural gefunden (vgl. H. H. Szmant et al, J. Chem. Tech Biotechnol. 31, 135 (1981)). Die Isolierung des gewünschten Produktes ist allerdings u. a. wegen des hohen Siedepunktes des Lösungsmittel schwierig und erfordert eine mehrstufige Extraktion.

In der DE-A-33 09 564 wird deshalb zur Isolierung des 5-Hydroxymethyl-furfural aus Dimethylsulfoxid enthaltenden Lösungen eine Derivatisierung zu 5-Acetoxy- methylfurfural vorgeschlagen. Dies erfordert neben einer Vakuumdestillation ausserdem zwei Reaktionsschritte (Acetylierung, Entacetylierung) und somit Zeit- und Chemikalienaufwand.

Mehrere Verfahren verwenden als Reaktionsmedium Gemische von Wasser und organischen Lösungsmitteln. In der US-A-2 929 823 wird Furfural zu wässrigen Saccharidlösungen zugegeben und kurzzeitig (0,1 - 120 s) auf 250 - 380°C erhitzt. Teerartige Nebenprodukte werden durch das zugegebene organische Lösungsmittel gelöst, ebenso wie 5-Hydroxymethyl-furfural. Die Reindarstellung des 5-Hydroxymethyl-furfurals erscheint dadurch nur schwierig durchführbar.

Ein weiteres zweiphasiges Verfahren ist in DE-A-30 33 527 beschrieben. Dabei werden unter milderen Bedingungen (unter 100°C) fructosehaltige wässrige Lösungen mit sauren Kationenaustauschern zersetzt, wobei ein organisches Lösungsmittel, das nicht mit Wasser mischbar ist, aber dennoch ein gutes Lösungsvermögen für 5-Hydroxymethyl-furfural besitzt, zugegen ist. Der große Nachteil dieses Verfahrens besteht darin, dass ein sehr großer Überschuss des organischen Lösungsmittels, bezogen auf die wässrige Phase (> 7:1), notwendig ist und die erforderlichen Lösungsmittel teuer und giftig sind. Außerdem macht die sehr gute Löslichkeit von 5-Hydroxymethyl-furfural in Wasser jegliche Extraktion des Produktes mit organischen Lösungsmitteln aus wässrigen Lösungen außerordentlich schwierig.

In der Veröffentlichung von C. Fayet et al, Carbohydr. Res. 122 (1983), 59, wird die Zersetzung von Sacchariden ohne Lösungsmittel, jedoch mit äquimolaren Katalysatormengen beschrieben. Der Katalysator Pyridinhydrochlorid ist jedoch für eine technische Anwendung des Verfahrens ungeeignet. Zudem schließt sich nach Zugabe von Wasser eine langwierige Extraktion (20 h) mit Ethylacetat an.

Dies kommt auch in der Veröffentlichung von D. W. Brown et al, J. Chem. Tech. Biotechnol. 32, 920 (1982) zum Ausdruck, wo es sinngemäß heißt, dass die neueren Methoden der Herstellung von 5-Hydroxymethyl-furfural den Nachteil hätten, dass das Produkt in wässriger Phase oder in einem polaren Lösungsmittel vorliegt, woraus die Isolierung schwierig sei.

In EP-A 0 230 250 ist ein Verfahren zur Herstellung von 5-Hydroxymethyl-furfural beschrieben, in dem Saccharide in wässriger Lösung, gegebenenfalls unter Zusatz von organischen Lösungsmitteln, über 100°C mit einem sauren Katalysator zersetzt werden. Die Reaktionsmischung wird nach Entfernung von gegebenenfalls vorhanden Lösungsmitteln unter ausschließlicher Verwendung von Wasser als Lösungsmittel über Ionenaustauschersäulen chromatographiert, eine bei größerer Aufgabemenge erhältliche Mischfraktion rechromatographiert und das 5-Hydroxymethyl-furfural aus den entsprechenden Fraktionen auskristallisiert. Zwar führt das beschriebene Verfahren angeblich zu 5-Hydroxymethyl-furfural in hoher Reinheit, aber das mehrfache Chromatographieren und Auskristallisieren ist in großtechnischem Maßstab zu aufwändig und teuer.

Weitere Verfahren zur Herstellung von 5-Hydroxymethyl-furfural sind beispielsweise in Bull. Soc. Chem. France 1987, 5, 855; J. Chem. Tech. Biotechnol. 1992, 55, 139; FR-A-2 669 635; FR-A-2 664 273; US-A-3,483,228; US-A-2,917,520; US-A-3,071,599; US-A-3,066,150 und US-A-2,750,394 offenbart.

Wie aus den obigen Beispielen hervorgeht, ist das größte Problem bei der Herstellung von 5-Hydroxymethyl-furfural, besonders im großtechnischen Maßstab, dieses Produkt von Ausgangsstoffen, Nebenprodukten und Lösungsmitteln abzutrennen. Aufgabe der vorliegenden Erfindung ist es daher, ein Verfahren zur Herstellung von 5-Hydroxymethyl-furfural bereitzustellen, bei dem das Produkt in hoher Reinheit erzeugt wird. Dabei soll das Herstellungsverfahren mit akzeptablen Ausbeuten an 5-Hydroxymethyl-furfural und wirtschaftlich durchführbar sein. Insbesondere bei der späteren Verwendung von 5-Hydroxymethyl-furfural als Arzneimittel ist entscheidend, dass bei der Herstellung keine stark toxischen Substanzen verwendet werden.

Gelöst wird die Aufgabe durch ein Verfahren zur Herstellung von 5-Hydroxymethyl-furfural über ein 5-Acyloxymethyl-furfural als Zwischenprodukt. Gegenstand der vorliegenden Erfindung ist daher sowohl ein Verfahren zur Herstellung von 5-Acyloxymethyl-furfural gemäß Formel (II) 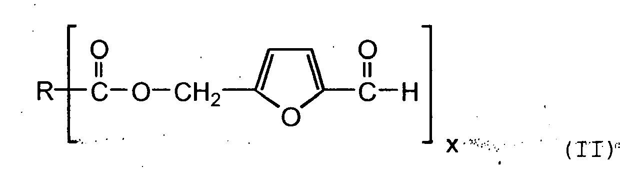 worin
x = 1 oder 2 ist,
R ein Alkyl-, Alkenyl-, Aryl- oder Heteroarylrest ist, wenn x = 1 ist,
R ein Alkylen-, Alkenylen-, Arylen- oder
Heteroarylenrest ist, wenn x = 2 ist,
aus einem Saccharid mit 6 Kohlenstoffatomen in der Monosaccharid-Einheit, umfassend die Schritte:
(a) Umsetzen des Saccharids mit(i) einer Säure, die kein saurer Ionenaustauscher ist, in Gegenwart eines Metallkations, ausgewählt aus Alkalimetallionen, Erdalkalimetallionen, Aluminiumionen und Mischungen daraus, oder (ii) einem sauren Ionenaustauscher in der H-Form in Gegenwart eines polaren aprotischen Lösungsmittels, und
   Abtrennen des sauren Ionenaustauschers, falls verwendet,
(b) Hinzufügen eines Anhydrids und/oder Chlorids einer Carbonsäure R-(-COOH)ₓ, worin R und x wie oben definiert sind, zu der Mischung aus Schritt (a) und reagieren lassen, um ein 5-Acyloxymethyl-furfural zu bilden, und
(c) Gewinnen des 5-Acyloxymethyl-furfurals,
   als auch die Weiterverarbeitung des 5-Acyloxymethyl-furfurals zu 5-Hydroxymethyl-furfural 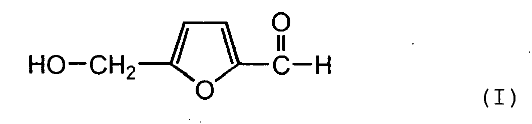 umfassend die weiteren Schritte:
(d) Lösen des 5-Acyloxymethyl-furfurals in einem Alkohol,
(e) Hinzufügen einer Base, ausgewählt aus (i)
   Alkalimetallcarbonaten und hydrogencarbonaten, Erdalkalimetallcarbonaten und -hydroxiden, basischen Salzen der Erdalkalimetalle, Aluminiumcarbonat, -hydroxid, -oxidhydroxid und basischen Salzen des Aluminiums, Mischungen der vorgenannten und (ii) basischen Ionenaustauschern in der OH-Form, zu der Lösung aus Schritt (d) und reagieren lassen, um 5-Hydroxymethyl-furfural zu bilden, und
(f) Gewinnen des 5-Hydroxymethyl-furfurals.

Ausgangsstoff der Herstellung von 5-Acyloxymethyl-furfural bzw. 5-Hydroxymethyl-furfural ist ein Saccharid mit 6 Kohlenstoffatomen in der Monosaccharid-Einheit. Grundsätzlich sind von dem Begriff "Saccharid" auch Mischungen aus verschiedenen Sacchariden umfasst. Auch können gegebenenfalls als Ausgangsstoffe Mischungen eingesetzt werden, die neben dem Saccharid noch andere Substanzen enthalten, solange diese das Verfahren nicht stören und das Saccharid in ausreichender Menge vorhanden ist. Mit dem Begriff "Saccharid" sind Monosaccharide, Oligosaccharide und Polysaccharide gemeint. Monosaccharide und Oligosaccharide werden auch als "Zucker" zusammengefasst. Die Monosaccharid-Einheit mit 6 Kohlenstoffatomen wird auch als "Hexose" bezeichnet, wobei hierin der Begriff "Hexose" sowohl die Aldohexosen als auch die Ketohexosen umfassen soll. Beispiele für als Ausgangsstoffe geeignete Monosaccharide sind Fructose, Glucose, Mannose, Allose, Altrose, Gulose, Idose, Galactose und Talose. Beispiele für als Ausgangsstoffe geeignete Oligosaccharide sind die Disaccharide, wie etwa Saccharose, Isomaltose, Gentiobiose, Melibiose, Trehalose, Mannopyranosyl-mannopyranose, Maltose, Milchzucker, Trehalose und Cellobiose, und Trisaccharide, wie etwa Raffinose. Beispiele für als Ausgangsstoffe geeignete Polysaccharide sind die Fructane (Polysaccharide mit 10 bis 40 Fructose-Einheiten), wie etwa Inulin, sowie Stärke und Celluose. Vorzugsweise ist das eingesetzte Saccharid ein Zucker oder eine Saccharidmischung, die Zucker enthält. Besonders bevorzugt ist das eingesetzte Saccharid ausgewählt ist aus D-Fructose in freier Form, einem Saccharid, das D-Fructose in gebundener Form enthält (z.B. Saccharose, Inulin), und einer Saccharidmischung, die D-Fructose in freier Form und/oder ein Saccharid, das D-Fructose in gebundener Form enthält, enthält.

Die erfindungsgemäße Herstellung von 5-Hydroxymethyl-furfural erfolgt über ein 5-Acyloxymethyl-furfural gemäß Formel (II) als Zwischenprodukt. In Formel (II) steht R für eine einwertige oder zweiwertige Gruppe. Wenn x = 1 ist, dann ist R eine Alkylgruppe, vorzugsweise C₁- bis C₂₀-Alkyl; eine Alkenylgruppe, vorzugsweise Ethenyl; eine Arylgruppe, vorzugsweise Phenyl, oder eine Heteroarylgruppe, wie etwa Pyridyl, Pyrimidyl, Pyrazinyl, Pyrrolyl, Furanyl oder Thiophenyl. Wenn x = 2 ist, dann ist R eine Alkylengruppe, vorzugsweise C₁- bis C₂₀-Alkylen; eine Alkenylengruppe, vorzugsweise Ethenylen; eine Arylengruppe, vorzugsweise Phenylen, oder eine Heteroarylengruppe, wie etwa Pyridylen, Pyrimidylen, Pyrazinylen, Pyrrolylen, Furanylen oder Thiophenylen. Besonders bevorzugt ist R gleich Methyl, d.h. 5-Acetoxymethyl-furfural wird als Zwischenprodukt hergestellt.

In Schritt (a) des erfindungsgemäßen Verfahrens wird das Saccharid entweder (i) mit einer Säure in Gegenwart eines Metallkations oder (ii) mit einem sauren Ionenaustauscher in der H-Form in Gegenwart eines polaren aprotischen Lösungsmittels umgesetzt. Es erfolgt u.a. die Dehydratisierung des Saccharids zu 5-Hydroxymethyl-furfural.

In Variante (i) kann die Säure irgendeine Säure sein, die sowohl das Saccharid als auch das Salz, das das Metallkation liefert, löst. Besonders gut geeignet sind Oxalsäure, Essigsäure und Salzsäure und deren Kombinationen, wobei Oxalsäure und Oxalsäure in Kombination mit Essigsäure bevorzugt sind . Neben der Säure wird in Schritt (a) (i) vorzugsweise kein weiteres Lösungsmittel zugegeben, d.h. die Säure wird vorzugsweise in weitgehend wasserfreier Form verwendet, wobei geringer Wassergehalte, die aus der technischen Herstellung der Säure resultieren mögen, die Reaktion nicht stören. Bei Variante (i) wird insbesondere vorzugsweise auf die Anwesenheit von später nur schwer abtrennbaren Lösungsmitteln, wie etwa Dimethylsulfoxid, verzichtet. Wird Essigsäure eingesetzt, so erfolgt bereits in Schritt (a) eine teilweise Acetylierung und 5-Acetoxymethyl-furfural entsteht.

Das Metallkation in Schritt (a) (i) ist ausgewählt aus Alkalimetallionen, Erdalkalimetallionen, Aluminiumionen und Mischungen daraus. Bevorzugt sind Mg²⁺-, Ca²⁺-, Na⁺- und Al³⁺-Ionen, besonders bevorzugt ist Mg²⁺. Das Metallkation wird vorteilhafterweise in Form eines festen Salzes zugegeben; gut geeignet sind die Chloride, z.B. MgCl₂, aber auch die Bromide, z.B. MgBr₂, und die Fluoride, z.B. MgF₂.

Ohne an diese Theorie gebunden sein zu wollen, wird angenommen, dass das Kation, z.B. Mg²⁺, die Furanoseform der Hexose, von der man glaubt, dass sie bei der Bildung der Furanverbindung eine wichtige Rolle spielt, in einem "Zucker-Kation-Komplex" stabilisiert und dadurch die Dehydratisierung katalysiert.

Gemäß einer bevorzugten Ausführungsform werden das Saccharid und das das Metallkation enthaltende Salz vorgelegt, gegebenenfalls erwärmt, und dann die Säure zugegeben. Prinzipiell ist aber auch jede andere Reihenfolge des Zusammengebens möglich.

Typischerweise werden das Saccharid und das Metallkation in einem Molverhältnis von 0,5:1 bis 2:1, bezogen auf die Monosaccharid-Einheit, eingesetzt, vorzugsweise von 1:1 bis 1,5:1, wobei die Verwendung von Saccharid und Metallkation in etwa äquimolaren Mengen besonders bevorzugt ist. Da die Säure in Variante (i) des Schritts (a) auch gleichzeitig als Lösungsmittel dient, wird sie üblicherweise in großem Überschuss eingesetzt, beispielsweise in 2- bis 20fachem molaren Überschuss, bezogen auf die Monosaccharid-Einheit.

In Schritt (a), Variante (ii) wird das Saccharid mit einem sauren Ionenaustauscher in der H-Form in Gegenwart eines polaren aprotischen Lösungsmittel umgesetzt und dieser vor Durchführung von Schritt (b) wieder abgetrennt, z.B. durch Filtration. Der saure Ionenaustauscher ist ein kationischer organischer oder anorganischer, üblicherweise organischer Ionenaustauscher. Bei einem organischen Ionenaustauscher handelt es sich um einen festen Polyelektrolyten, meist in Körnerform, mit einem dreidimensionalen, wasserunlöslichen hochmolekularen Polymergerüst (Matrix), in das zahlreiche geladene "Ankergruppen" eingebaut sind. Deren locker gebundene Gegenionen können gegen andere gleichsinnig geladene Ionen ausgetauscht werden. Als Matrixharze kommen überwiegend Styrolcoplymere, vorzugsweise mit Divinylbenzol vernetztes Polystyrol, und Acrylpolymere, wie etwa die Acrylat-, Methacrylat- und Acrylnitrilcoplymere, die jeweils mit Divinylbenzol vernetzt sind, zum Einsatz. Bei einem sauren Ionenaustauscher in der H-Form sind die Ankergruppen saure Gruppen in ihrer protonierten Form (Säureform), d.h. die austauschbaren Gegenionen sind H⁺. In Schritt (a) (i) der vorliegenden Erfindung wird vorzugsweise ein stark saurer Kationenaustauscher in der H-Form verwendet, der Sulfonsäuregruppen in ihrer protonierten Form als Ankergruppen trägt. Es ist auch der Einsatz von schwach sauren Kationenaustauschern, die Carbonsäuregruppen in ihrer protonierten Form tragen, möglich; allerdings wird sich in diesem Fall die Reaktionszeit verlängern. Besonders bevorzugt ist ein Styrol-Divinylbenzol-Copolymerharz mit protonierten Sulfonsäuregruppen als Ankergruppen.

Typischerweise wird der saure Ionenaustauscher in einer Menge eingesetzt, die einem großen molaren Überschuss der sauren Ankergruppen bezogen auf Monosaccharid-Einheit des eingesetztes Saccharid, vorzugsweise einem 2- bis 20fachen, bevorzugter 10- bis 20fachen molaren Überschuss, entspricht. Die Anzahl der sauren Ankergruppen und damit die benötigte Menge des Ionenaustauschers berechnet sich aus der Austauscherkapazität, die üblicherweise in Äquivalent/l oder Äquivalent/kg angegeben wird.

Beispiele für in Schritt (a) (ii) geeignete polare aprotische Lösungsmittel umfassen Dimethylsulfoxid (DMSO), N-Methylpyrrolidon (NMP), Dimethylformamid, N,N-Dimethylacetamid, Acetonitril und deren Mischungen. Bevorzugt sind DMSO und NMP, wobei NMP besonders bevorzugt ist.

Typischerweise werden das Saccharid und das polare aprotische Lösungsmittel in einem Molverhältnis von 1:2 bis 1:20, vorzugsweise von 1:2 bis 1:10, eingesetzt.

In einer bevorzugten Ausführungsform wird Schritt (a) bei einer Temperatur von 80 bis 140°C, bevorzugter von 80 bis 110°C, bei einer Reaktionsdauer von 4 bis 7 h, bevorzugter von 4 bis 6 h, durchgeführt. Bei entsprechend längeren Reaktionszeiten können auch niedrigere Temperaturen angewandt werden. Nach oben ist die Temperatur durch den Siedepunkt des Reaktionsgemisches begrenzt.

In Schritt (b) wird ein Anhydrid und/oder Chlorid einer Carbonsäure R-(-COOH)ₓ, worin R und x wie oben definiert sind, zu der Mischung aus Schritt (a) gegeben und es erfolgt die Acylierung zu einem 5-Acyloxymethyl-furfural. Wie oben bereits erwähnt, ist R vorzugsweise Methyl, d.h. in Schritt (b) wird vorzugsweise Essigsäureanhydrid und/oder Acetylchlorid zugegeben, wobei Essigsäureanhydrid besonders bevorzugt ist, und 5-Acetoxymethyl-furfural wird gebildet. Wurde in Schritt (a) mit Essigsäure angesäuert und war so bereits in diesem ersten Schritt eine teilweise Acetylierung erfolgt, so wird nun in Schritt (b) die Acetylierung durch die Zugabe von Essigsäureanhydrid und/oder Acetylchlorid vervollständigt.

Weitere beispielhafte Anhydride und Chloride, die in Schritt (b) der vorliegenden Erfindung verwendet werden können, ergeben sich unmittelbar aus den zuvor genannten Beispielen für die Gruppe R. Es versteht sich, dass, wenn ein 5-Acyloxymethyl-furfural mit x = 2 erhalten werden soll, die entsprechenden Dicarbonsäuren eingesetzt werden müssen. Viele Dicarbonsäuren bilden aufgrund ihrer Molekülstruktur cyclische Anhydride. Gut geeignet sind etwa die preiswerten Anhydride der Malein-, Fumar-, Pyridyl-2,3-dicarbon- und Pyrazin-2,3-dicarbonsäure.

In einer bevorzugten Ausführungsform der vorliegenden Erfindung wird Schritt (b) in Anwesenheit eines basischen Katalysators, z.B. 4-(N,N-Dimethylamino)-pyridin, durchgeführt. Der basische Katalysator wird üblicherweise in katalytischen Mengen zugegeben, etwa in einem Molverhältnis von Katalysator zu Monosaccharid-Einheit von 1:10 bis 1:100.

Aufgrund des entstehenden Reaktionswassers werden bei Variante (i) des vorliegenden Verfahrens das Anhydrid und/oder Chlorid der Carbonsäure R-(-COOH)ₓ typischerweise in einem großen molaren Überschuss zugegebenen. Vorzugsweise wird ein Äquivalentverhältnis von Monosaccharid-Einheit des zu Beginn eingesetzten Saccharids zu Anhydrid und/oder Chlorid der Carbonsäure R-(-COOH)ₓ von mindestens 1:4, bevorzugter von 1:4 bis 1:12 verwendet.

Bei Variante (ii) des vorliegenden Verfahrens werden die Monosaccharid-Einheit und das Anhydrid und/oder Chlorid der Carbonsäure R-(-COOH)ₓ typischerweise in einem Äquivalentverhältnis von 1:0,7 bis 1:3, vorzugsweise von 1:0,8 bis 1:1,2 eingesetzt, wobei die Verwendung der Monosaccharid-Einheit und des Anhydrids und/oder Chlorids der Carbonsäure R-(-COOH)ₓ in etwa äquivalenten Mengen (d.h. äquimolaren Mengen für x = 1) besonders bevorzugt ist. Ohne an diese Theorie gebunden sein zu wollen, wird vermutet, dass das entstehende Reaktionswasser an den Ionenaustauscher gebunden wird.

Vorzugsweise erfolgt Schritt (b) unter Rühren.

Typischerweise wird Schritt (b) bei einer Temperatur von 0 bis 40°C bei einer Reaktionsdauer von 0,5 bis 3 h durchgeführt. Bei entsprechend längeren Reaktionszeiten können auch niedrigere Temperaturen angewandt werden. Da die Acylierungsreaktion exotherm ist, muss gegebenenfalls gekühlt werden. Nach oben ist die Temperatur durch den Siedepunkt des Reaktionsgemisches begrenzt.

Um das Gleichgewicht der Acylierungsreaktion in Schritt (b) nach rechts zu verschieben, ist es vorteilhaft, die entstehende Carbonsäure R-(-COOH)ₓ aus der Mischung abzutrennen. Dies kann durch übliche Maßnahmen erfolgen, z. B. durch Abdestillieren, gegebenenfalls unter Zusatz eines aprotischen nichtwassermischbaren organischen Lösungsmittels, wie etwa Toluol, Xylol, tert.-Butylmethylether (MtBE), Methylisobutylketon (MIBK), besonders bevorzugt MtBE. Man erhält in diesem Falle 5-Acyloxymethyl-furfural gelöst in dem organischen Lösungsmittel. Wurde Schritt (a) des erfindungsgemäßen Verfahren nach Variante (ii) durchgeführt, d.h. in Anwesenheit eines polaren aprotischen Lösungsmittels, so wird dieses nun in Schritt (b) vorzugsweise zum großen Teil mit abdestilliert. Auch dann kann die Destillation unter Zusatz der oben genannten aprotischen nichtwassermischbaren organischen Lösungsmittel erfolgen.

In anschließendem Schritt (c) wird das 5-Acyloxymethyl-furfural gewonnen, d.h. von den unerwünschten Nebenprodukten, nicht umgesetzten Ausgangsprodukten und gegebenenfalls Lösungsmittel befreit. Die Reinigung des 5-Acyloxymethyl-furfurals erfolgt nach in der Technik üblichen Methoden, z. B. durch Extraktion, Filtration, Destillation und deren Kombinationen. Zur Extraktion gibt man Wasser und ein aprotisches nichtwassermischbares organisches Lösungsmittel wie oben beschrieben zu, falls letzteres nicht bereits in Schritt (b) zugegeben wurde. Vorteilhaft ist ferner die Zugabe von Aktivkohle, um daran polymere Nebenprodukte, die weder in Wasser noch in dem organischen Lösungsmittel löslich sind, zu binden. Die Aktivkohle kann dann zusammen mit den festen Nebenprodukten, gegebenenfalls unter Zugabe einer Filterhilfe, wie etwa Celite®, abfiltriert werden. Die wässrige Phase wird dann von der organischen Phase, in der sich das gewünschte Produkt 5-Acyloxymethyl-furfural befindet abgetrennt, das organische Lösungsmittel abdestilliert und das verbleibende 5-Acyloxymethyl-furfural zur Feinreinigung destilliert, vorzugsweise im Vakuum. Die Art und Weise der Aufbereitung des 5-Acyloxymethyl-furfural-Zwischenprodukts ist jedoch für die vorliegende Erfindung nicht wesentlich und es ist auch jedes andere geeignete Verfahren zur Gewinnung des 5-Acyloxymethyl-furfurals möglich.

Typischerweise betragen die Ausbeuten an 5-Acyloxymethyl-furfural, bezogen auf Saccharid, 20 bis 80 %, vorzugsweise 30 bis 80 %, bevorzugter 40 bis 80 % und am meisten bevorzugt 42 bis 80 %.

Soll das 5-Acyloxymethyl-furfural zu 5-Hydroxymethyl-furfural weiterverarbeitet werde, so wird es in Schritt (d) in einem Alkohol, vorzugsweise einem aliphatischen primären oder sekundäre Alkohole, wie etwa Methanol, Ethanol, Propanol, Butanol, Pentanol, Hexanol, Isopropanol oder Isobutanol, oder Benzylalkohol gelöst, gegebenenfalls unter Anwendung von Wärme. Besonders bevorzugt wird Methanol als Alkohol verwendet, in dem sich beispielsweise 5-Acetoxymethyl-furfural bereits bei Raumtemperatur (ca. 20 - 25°C) leicht löst. Typischerweise wird das 5-Acyloxymethyl-furfural im Alkohol in einem Molverhältnis von 1:5 bis 1:10 gelöst.

Dann wird in Schritt (e) eine Base, ausgewählt aus (i) Alkalimetallcarbonaten und hydrogencarbonaten, Erdalkalimetallcarbonaten und -hydroxiden, basischen Salzen der Erdalkalimetalle, Aluminiumcarbonat, -hydroxid, -oxidhydroxid und basischen Salzen des Aluminiums, Mischungen der vorgenannten und (ii) basischen Ionenaustauschern in der OH-Form, zugegeben und durch Hydrolyse (Verseifung) der Acyloxygruppe (Estergruppe) entsteht das gewünschte Produkt 5-Hydroxymethyl-furfural. Bevorzugte Basen sind ein basischer Ionenaustauscher in der OH-Form, Kaliumcarbonat, Natriumcarbonat und Calciumcarbonat, wobei ein basischer Ionenaustauscher in der OH-Form und Kaliumcarbonat besonders bevorzugt ist.

Der basische Ionenaustauscher ist ein anionischer organischer oder anorganischer, üblicherweise organischer Ionenaustauscher. Organische Ionenaustauscher und ihre Matrixpolymere wurden bereits zuvor allgemein beschrieben. Bei einem basischen Ionenaustauscher in der OH-Form sind die Ankergruppen positiv geladene Gruppen und die austauschbaren Gegenionen sind OH⁻. In Schritt (e) der vorliegenden Erfindung wird vorzugsweise ein stark basischer Anionenaustauscher verwendet, der quartäre Ammoniumgruppen, insbesondere Benzyltrimethylammoniumgruppen (bekannt als "Typ 1") oder Benzyldimethylethanolammoniumgruppen (bekannt als "Typ 2"), als Ankergruppen trägt und Hydroxidionen als Gegenionen enthält. Es können auch schwächer basische Anionenaustauscher mit tertiären Aminogruppen eingesetzt werden. Besonders bevorzugt ist ein Styrol-Divinylbenzol-Copolymerharz mit Benzyltrimethylammoniumgruppen als Ankergruppen in seiner OH-Form, z.B. AMBERLYST® A26 OH, erhältlich von Rohm and Haas Company, Philadelphia, U.S.A.

In beiden Varianten des Schritts (e) werden das 5-Acyloxymethyl-furfural und die Base typischerweise in einem Äquivalentverhältnis von 1:0,9 bis 1:3 eingesetzt, vorzugsweise von 1:1 bis 1:2, bevorzugter von 1:1 bis 1:1,5, wobei etwa äquivalente Mengen der beiden Reaktanten besonders bevorzugt sind. Bei Verwendung eines basischen Ionenaustauschers errechnet sich dessen benötigte Menge unter Berücksichtigung der zuvor genannten Aquivalentverhältnisse aus der Austauscherkapazität, die üblicherweise in Äquivalent/l oder Äquivalent/kg angegeben wird.

Die Hydrolyse in Schritt (e) erfolgt vorzugsweise bei relativ niedriger Temperatur (ca. 20 - 35°C) bei einer Reaktionsdauer von 1 bis 3 h. Je nach Art des Acylrests des 5-Acyloxymethyl-furfurals können jedoch auch höhere Temperaturen angewandt werden.

In anschließendem Schritt (f) wird das 5-Hydroxymethyl-furfural gewonnen, d.h. von den unerwünschten Nebenprodukten, nicht umgesetzten Ausgangsprodukten, festem Ionenaustauscher, falls verwendet, und Lösungsmittel befreit. Die Reinigung des 5-Hydroxymethyl-furfurals erfolgt nach in der Technik üblichen Methoden, z. B. Filtration, Destillation und/oder Kristallisation. Vorzugsweise filtriert man zuerst die festen Nebenprodukte und, falls verwendet, den Ionenaustauscher ab und destilliert dann das Filtrat, um das 5-Hydroxymethyl-furfural rein zu gewinnen. Da 5-Hydroxymethyl-furfural temperaturempfindlich ist und u.a. zur Polymer- und Etherbildung neigt, empfiehlt es sich, eine Molekulardestillation oder Kurzwegdestillation im Vakuum, z.B. mithilfe eines Kurzwegverdampfers, Fallfilmverdampfers oder Dünnschichtverdampfers, durchzuführen. In einer anderen bevorzugten Ausführungsform wird das 5-Hydroxymethyl-furfural nach Filtration durch Kristallisation aus einem geeigneten Lösungsmittel, wie etwa Diethylether, Diisopropylether, MtBE oder tert.-Butylethylether (EtBE), vorzugsweise MtBE, bei geeigneter Temperatur gewonnen.

Typischerweise beträgt die Ausbeute an 5-Hydroxymethyl-furfural, bezogen auf Saccharid, 10 bis 70 %, vorzugsweise 20 bis 70 %, bevorzugter 25 bis 70 % und am meisten bevorzugt 30 bis 70 %.

Typischerweise wird nach dem erfindungsgemäßen Verfahren 5-Hydroxymethyl-furfural in einer Reinheit von mindestens 90 %, vorzugsweise mindestens 95 %, bevorzugter mindestens 98 % und am meistens bevorzugt mindestes 99 % erzeugt. Damit liegt die Reinheit über der von mit vergleichbaren, chromatographiefreien Verfahren erreichbaren Reinheit. Die hohe erzielbare Reinheit macht das erfindungsgemäß hergestellte 5-Hydroxymethyl-furfural für kosmetische oder pharmazeutische Zwecke geeignet.

In der vorliegenden Erfindung wird zum ersten Mal ein Verfahren zur Herstellung von 5-Hydroxymethyl-furfural zur Verfügung gestellt, das eine großtechnische Produktion von 5-Hydroxymethyl-furfural in hoher Reinheit ermöglicht

Die Erfindung soll nun anhand von Ausführungsbeispielen weiter veranschaulicht werden.

### Beispiel 1A: Herstellung von 5-Acetoxymethyl-furfural unter Verwendung von MgCl₂

107 g (0,6 mol) D-Fructose und 122 g (0,6 mol) MgCl₂·6H₂0 werden in einen 2-1-Dreihalskolben unter Rühren eingetragen und für 30 min auf 75°C erwärmt. Das Reaktionsgemisch wird mit 420 ml wasserfreier Essigsäure (Eisessig) versetzt und 4 h lang auf 90-95°C erhitzt. Danach destilliert man ca. 80 % der Essigsäure ab, kühlt auf Raumtemperatur und gibt 5 g (0,04 mol) 4-(N,N-Dimethylamino)-pyridin hinzu. Unter Rühren tropft man bei 30 - 40°C 616 ml (6,5 mol) Essigsäureanhydrid zum Reaktionsgemisch und destilliert die 600 - 700 ml Essigsäure ab. Das Reaktionsgemisch wird bei ca. 80°C langsam mit 500 ml Wasser, 500 ml MIBK und 50 g Aktivkohle versetzt. Nach der Filtration über einen Druckfilter werden die organische und wässrige Phase getrennt. Die organische Phase wird am Rotationsverdampfer vom Lösungsmittel befreit und der Rückstand im Vakuum bei 117-125°C/7 mbar destilliert. Ausbeute: 45,9 g (0,27 mol, 45 %) 5-Acetoxymethyl-furfural

### Beispiel 1B: Verseifung von 5-Acetoxymethyl-furfural unter Verwendung von Kaliumcarbonat

179,1 g (1,07 mol) des in Beispiel 1A hergestellten 5-Acetoxymethyl-furfural werden in 1,1 l Methanol gelöst und unter Rühren mit 140 g (1,01 mol) Kaliumcarbonat bei 20-25°C versetzt. Nach 1 h wird das Reaktionsgemisch mit 10 g Aktivkohle versetzt, 20 min gerührt, die festen Bestandteile abfiltriert und mit 100 ml MeOH nachgewaschen. Die klare methanolische Lösung wird im Vakuum am Rotationsverdampfer eingeengt. Der Rückstand wird mit 300 ml MtBE versetzt. Die ausgefallenen Salze werden abfiltriert und mit 20 ml MtBE nachgewaschen. Die Lösung wird am Rotationsverdampfer eingeengt und der Rückstand wird in einem Kurzwegverdampfer bei 90°C/0.03 mbar destilliert. Ausbeute: 85,2 g (0,68 mol, 63 % bezogen auf 5-Acetoxymethyl-furfural, 29 % bezogen auf D-Fructose). Alternativ kann der Rückstand auch mit MtBE versetzt und das Produkt bei 0°C auskristallisiert werden (Ausbeute: 98,4 g (0,78 mol, 73 % bezogen auf 5-Acetoxymethyl-furfural, 33 % bezogen auf D-Fructose)).

### Beispiel 2A: Herstellung von 5-Acetoxymethyl-furfural unter Verwendung eines sauren Ionenaustauschers

In 90 l NMP wurden 39,4 kg (219 mol) D-Fructose und 5,9 kg getrockneter saurer Ionenaustauscher Dowex® 50WX8-200 (Styrol-Divinylbenzol-Copolymerharz mit SO₃H-Gruppen, erhältlich von The Dow Chemical Company, Midland, U.S.A.) in der H-Form unter Rühren eingetragen und für 6 h auf 110°C erhitzt. Das Reaktionsgemisch wird nach dem Abkühlen filtriert und mit 8 l NMP nachgewaschen. Das Filtrat wird unter Rühren mit 390 g (3,2 mol) (4-(N,N-Dimethylamino)-pyridin und 20,5 l (217 mol) Essigsäureanhydrid bei 20-25°C innerhalb 1 h versetzt. Nach 1 h Nachreaktion wird die braune Reaktionsmischung im Vakuum (von 50 - 10 mbar) vom Lösungsmittel bei 90-100°C befreit. Der Rückstand wird nach dem Abkühlen mit 160 l MtBE, 60 l Wasser und 4 kg Aktivkohle versetzt. Die Suspension wird über Celite® filtriert. Nach der Phasentrennung wird das Lösungsmittel des Filtrates im Vakuum (20 mbar) bei 50°C abdestilliert und der Rückstand wird im Vakuum bei 106-110°C/5 mbar fraktioniert destilliert.
Ausbeute: 15,5 kg (92 mol, 42 %) 5-Acetoxymethyl-furfural

### Beispiel 2B: Verseifung von 5-Acetoxymethyl-furfural unter Verwendung eines basischen Ionenaustauschers

10,9 kg (65 mol) 5-Acetoxymethyl-furfural werden in 60 l Methanol gelöst und unter Rühren mit 1,1 kg getrocknetem stark basischen Ionenaustauscher Amberlyst® A26 OH (Styrol-Divinylbenzol-Copolymerharz mit quartären Ammoniumgruppen (Typ 1) in OH-Form, erhältlich von Rohm and Haas Company, Philadelphia, U.S.A.) bei 25 - 30°C versetzt. Nach 1 h wird das Reaktionsgemisch mit 1 kg Aktivkohle versetzt, 60 min gerührt, über Celite® filtriert und mit 10 l Methanol nachgewaschen. Die klare methanolische Lösung wird im Vakuum bei maximal 40°C eingeengt. Der Rückstand wird mit 8 l MtBE versetzt und langsam auf 5°C abgekühlt. Das ausgefallene Produkt wird abgesaugt, mit 1,5 l eiskaltem MtBE nachgewaschen und im Vakuum bei 20°C getrocknet. Ausbeute: 7,01 kg (56 mol, 86 % bezogen auf 5-Acetoxymethyl-furfural, 36 % bezogen auf D-Fructose) 5-Hydroxymethyl-furfural. Alternativ kann der Rückstand in einem Kurzwegverdampfer bei 90°C/0,03 mbar destilliert werden (Ausbeute: 5,42 kg (43 mol, 67 % bezogen auf 5-Acetoxymethyl-furfural, 28 % bezogen auf D-Fructose)).

Die Reinheit des hergestellten 5-Hydroxymethyl-furfurals ist in allen Beispielen > 99 % wie mithilfe von HPLC und NMR bestimmt wurde.

## Patentansprüche

1. Verfahren zur Herstellung eines 5-Acyloxymethyl-furfurals der Formel (II) 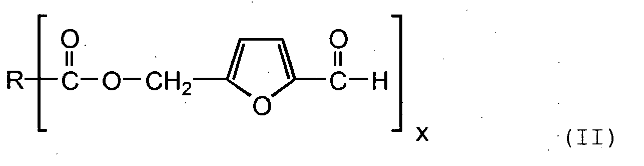 worin
x = 1 oder 2 ist,
R ein Alkyl-, Alkenyl-, Aryl- oder Heteroarylrest ist, wenn x = 1 ist,
R ein Alkylen-, Alkenylen-, Arylen- oder Heteroarylenrest ist, wenn x = 2 ist,
aus einem Saccharid mit 6 Kohlenstoffatomen in der Monosaccharid-Einheit, umfassend die Schritte:
(a) Umsetzen des Saccharids mit
(i) einer Säure, die kein saurer Ionenaustauscher ist, in Gegenwart eines Metallkations, ausgewählt aus Alkalimetallionen, Erdalkalimetallionen, Aluminiumionen und Mischungen daraus, oder (ii) einem sauren Ionenaustauscher in der H-Form in Gegenwart eines polaren aprotischen Lösungsmittels, und
Abtrennen des sauren Ionenaustauschers, falls verwendet,
(b) Hinzufügen eines Anhydrids und/oder Chlorids einer Carbonsäure R-(-COOH)ₓ, worin R und x wie oben definiert sind, zu der Mischung aus Schritt (a) und reagieren lassen, um ein 5-Acyloxymethylfurfural zu bilden, und
(c) Gewinnen des 5-Acyloxymethyl-furfurals.

2. Verfahren nach Anspruch 1,
**dadurch gekennzeichnet,dass** R gleich Methyl ist.

3. Verfahren nach Anspruch 2,
**dadurch gekennzeichnet, dass** in Schritt (b) Essigsäureanhydrid zugesetzt wird.

4. Verfahren nach einem der vorherigen Ansprüche,
**dadurch gekennzeichnet, dass** die Säure in Schritt (a)(i) ausgewählt ist aus Oxalsäure, Essigsäure, Salzsäure und deren Mischungen.

5. Verfahren nach einem der vorherigen Ansprüche,
**dadurch gekennzeichnet, dass** das Metallkation in Schritt (a) (i) Mg²⁺ ist.

6. Verfahren nach einem der vorherigen Ansprüche,
**dadurch gekennzeichnet,dass** das in Schritt (a) eingesetzte Saccharid ein Zucker oder eine Saccharidmischung, die Zucker enthält, ist.

7. Verfahren nach einem der vorherigen Ansprüche,
**dadurch gekennzeichnet,dass** das in Schritt (a) eingesetzte Saccharid ausgewählt ist aus D-Fructose in freier Form, einem Saccharid, das D-Fructose in gebundener Form enthält, und einer Saccharidmischung, die D-Fructose in freier Form und/oder ein Saccharid, das D-Fructose in gebundener Form enthält, enthält.

8. Verfahren nach einem der vorherigen Ansprüche,
**dadurch gekennzeichnet, dass** das polare aprotische Lösungsmittels in Schritt (a)(ii) Dimethylsulfoxid oder N-Methylpyrrolidon ist.

9. Verfahren nach einem der vorherigen Ansprüche,
**dadurch gekennzeichnet, dass** Schritt (b) in Gegenwart eines basischen Katalysators erfolgt.

10. Verfahren nach einem der vorherigen Ansprüche,
**dadurch gekennzeichnet, dass** der basische Katalysator 4-(N,N-Dimethylamino)-pyridin ist.

11. Verfahren zur Herstellung von 5-Hydroxymethyl-furfural 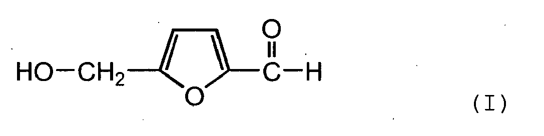 umfassend die Herstellung eines 5-Acyloxymethyl-furfurals nach einem der Ansprüche 1 bis 10 und die weiteren Schritte:
(d) Lösen des 5-Acyloxymethyl-furfurals in einem Alkohol,
(e) Hinzufügen einer Base, ausgewählt aus (i) Alkalimetallcarbonaten und hydrogencarbonaten, Erdalkalimetallcarbonaten und -hydroxiden, basischen Salzen der Erdalkalimetalle, Aluminiumcarbonat, -hydroxid, -oxidhydroxid und basischen Salzen des Aluminiums, Mischungen der vorgenannten und (ii) basischen Ionenaustauschern in der OH-Form, zu der Lösung aus Schritt (d) und reagieren lassen, um 5-Hydroxymethyl-furfural zu bilden, und
(f) Gewinnen des 5-Hydroxymethyl-furfurals.

12. Verfahren nach Anspruch 11,
**dadurch gekennzeichnet, dass** der Alkohol zum Lösen des 5-Hydroxymethyl-furfurals in Schritt (d) Methanol ist.

13. Verfahren nach Anspruch 11 oder 12,
**dadurch gekennzeichnet, dass** die Base in Schritt (e) Kaliumcarbonat ist.

14. Verfahren nach einem der Ansprüche 11 bis 13,
**dadurch gekennzeichnet, dass** die Gewinnung des 5-Hydroxymethyl-furfurals in Schritt (f) eine Kurzwegdestillation des 5-Hydroxymethyl-furfurals umfasst.

15. Verfahren nach einem der Ansprüche 11 bis 13,
**dadurch gekennzeichnet, dass** die Gewinnung des 5-Hydroxymethyl-furfurals in Schritt (f) die Kristallisation des 5-Hydroxymethyl-furfurals aus einem geeigneten Lösungsmittel umfasst.

## Claims

1. Process for preparing a 5-acyloxymethylfurfural of the formula (II) 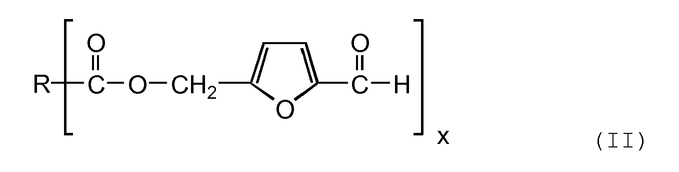 in which
x = 1 or 2,
R is an alkyl, alkenyl, aryl or heteroaryl radical when x = 1,
R is an alkylene, alkenylene, arylene or heteroarylene radical when x = 2,
from a saccharide having 6 carbon atoms in the monosaccharide unit, comprising the steps of:
(a) reacting the saccharide with
(i) an acid which is not an acidic ion exchanger in the presence of a metal cation selected from alkali metal ions, alkaline earth metal ions, aluminium ions or mixtures thereof, or (ii) an acidic ion exchanger in the H form in the presence of a polar aprotic solvent, and
removing the acidic ion exchanger, if used,
(b) adding an anhydride and/or chloride of a carboxylic acid R-(-COOH)ₓ in which R and x are each as defined above to the mixture from step (a) and allowing them to react in order to form a 5-acyloxymethylfurfural, and
(c) recovering the 5-acyloxymethylfurfural.

2. Process according to Claim 1,
**characterized in that** R is methyl.

3. Process according to Claim 2,
**characterized in that** acetic anhydride is added in step (b).

4. Process according to one of the preceding claims,
**characterized in that** the acid in step (a) (i) is selected from oxalic acid, acetic acid, hydrochloric acid and mixtures thereof.

5. Process according to one of the preceding claims,
**characterized in that** the metal cation in step (a) (i) is Mg²⁺.

6. Process according to one of the preceding claims,
**characterized in that** the saccharide used in step (a) is a sugar or a saccharide mixture which comprises sugars.

7. Process according to one of the preceding claims,
**characterized in that** the saccharide used in step (a) is selected from D-fructose in free form, a saccharide which contains D-fructose in bound form, and a saccharide mixture which contains D-fructose in free form and/or a saccharide which contains D-fructose in bound form.

8. Process according to one of the preceding claims,
**characterized in that** the polar aprotic solvent in step (a)(ii) is dimethyl sulphoxide or N-methylpyrrolidone.

9. Process according to one of the preceding claims,
**characterized in that** step (b) is effected in the presence of a basic catalyst.

10. Process according to one of the preceding claims,
**characterized in that** the basic catalyst is 4-(N,N-dimethylamino)pyridine.

11. Process for preparing 5-hydroxymethylfurfural 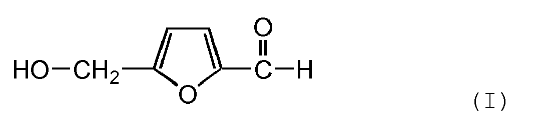 comprising the preparation of a 5-acyloxymethylfurfural according to one of Claims 1 to 10 and the further steps of:
(d) dissolving the 5-acyloxymethylfurfural in an alcohol,
(e) adding a base selected from (i) alkali metal carbonates and hydrogencarbonates, alkaline earth metal carbonates and hydroxides, basic salts of the alkaline earth metals, aluminium carbonate, aluminium hydroxide, aluminium oxide hydroxide, and basic salts of aluminium, mixtures of the above and (ii) basic ion exchangers in the OH form, to the solution from step (d) and allowing them to react in order to form 5-hydroxymethylfurfural, and
(f) recovering the 5-hydroxymethylfurfural.

12. Process according to Claim 11,
**characterized in that** the alcohol for dissolving the 5-hydroxymethylfurfural in step (d) is methanol.

13. Process according to Claim 11 or 12,
**characterized in that** the base in step (e) is potassium carbonate.

14. Process according to one of Claims 11 to 13,
**characterized in that** the recovery of the 5-hydroxymethylfurfural in step (f) comprises a short-path distillation of the 5-hydroxymethylfurfural.

15. Process according to one of Claims 11 to 13,
**characterized in that** the recovery of the 5-hydroxymethylfurfural in step (f) comprises the crystallization of the 5-hydroxymethylfurfural from a suitable solvent.

## Revendications

1. Procédé pour la préparation d'un 5-acyloxyméthyl-furfural de formule (II) 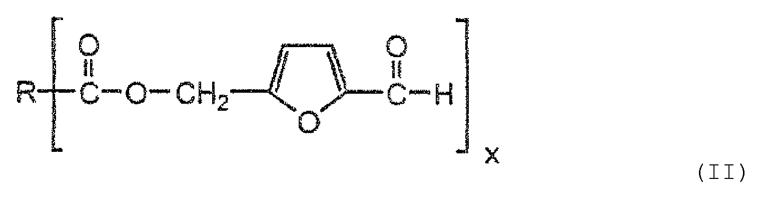 dans laquelle
x = 1 ou 2,
R est un radical alkyle, alcényle, aryle ou hétéroaryle, lorsque x = 1,
R est un radical alkylène, alcénylène, arylène ou hétéroarylène, lorsque x = 2,
à partir d'un saccharide ayant 6 atomes de carbone dans l'unité monosaccharide, comprenant les étapes :
(a) mise en réaction du saccharide avec
(i) un acide, qui n'est pas un échangeur d'ions acide, en présence d'un cation métallique choisi parmi des ions de métaux alcalins, des ions de métaux alcalino-terreux, des ions aluminium et des mélanges de ceux-ci, ou (ii) un échangeur d'ions acide sous la forme H, en présence d'un solvant aprotique polaire, et
séparation de l'échangeur d'ions acide, s'il est utilisé,
(b) addition d'un anhydride et/ou d'un chlorure d'un acide carboxylique R-(-COOH)ₓ, dans lequel R et x sont tels que définis ci-dessus, au mélange provenant de l'étape (a) et mise en réaction pour la formation d'un 5-acyloxyméthyl-furfural, et
(c) récupération du 5-acyloxyméthyl-furfural.

2. Procédé selon la revendication 1, **caractérisé en ce que** R est le groupe méthyle.

3. Procédé selon la revendication 2, **caractérisé en ce que** dans l'étape (b) on ajoute de l'anhydride acétique.

4. Procédé selon l'une quelconque des revendications précédentes, **caractérisé en ce que** l'acide dans l'étape (a)(i) est choisi parmi l'acide oxalique, l'acide acétique, l'acide chlorhydrique et des mélanges de ceux-ci.

5. Procédé selon l'une quelconque des revendications précédentes, **caractérisé en ce que** le cation métallique dans l'étape (a)(i) est Mg²⁺.

6. Procédé selon l'une quelconque des revendications précédentes, **caractérisé en ce que** le saccharide utilisé dans l'étape (a) est un sucre ou un mélange de saccharides qui contient un sucre.

7. Procédé selon l'une quelconque des revendications précédentes, **caractérisé en ce que** le saccharide choisi dans l'étape (a) est choisi parmi le D-fructose sous forme libre, un saccharide qui contient du D-fructose sous forme liée, et un mélange de saccharides qui contient du D-fructose sous forme libre et/ou un saccharide qui contient du D-fructose sous forme liée.

8. Procédé selon l'une quelconque des revendications précédentes, **caractérisé en ce que** le solvant aprotique polaire dans l'étape (a)(ii) est le diméthylsulfoxyde ou la N-méthylpyrrolidone.

9. Procédé selon l'une quelconque des revendications précédentes, **caractérisé en ce que** l'étape (b) s'effectue en présence d'un catalyseur basique.

10. Procédé selon l'une quelconque des revendications précédentes, **caractérisé en ce que** le catalyseur basique est la 4-(N,N-diméthylamino)-pyridine.

11. Procédé pour la préparation de 5-hydroxyméthyl-furfural 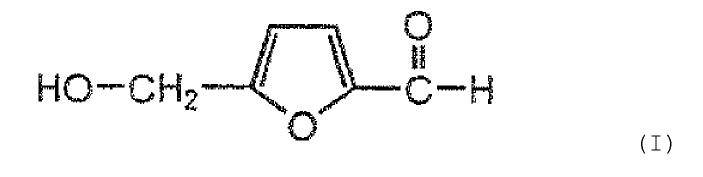 comprenant la préparation d'un 5-acyloxyméthyl-furfural selon l'une quelconque des revendications 1 à 10 et les autres étapes :
d) dissolution du 5-acyloxyméthyl-furfural dans un alcool,
e) addition d'une base, choisie parmi (i) des carbonates et hydrogénocarbonates de métaux alcalins, des carbonates et hydroxydes de métaux alcalino-terreux, des sels basiques des métaux alcalino-terreux, le carbonate, l'hydroxyde, l'oxyde-hydroxyde d'aluminium et des sels basiques de l'aluminium, des mélanges des composés précités et (ii) des échangeurs d'ions basiques sous la forme OH, à la solution provenant de l'étape (d) et mise en réaction, pour la formation de 5-hydroxyméthyl-furfural, et
(f) récupération du 5-hydroxyméthyl-furfural.

12. Procédé selon la revendication 11, **caractérisé en ce que** l'alcool pour la dissolution du 5-hydroxyméthyl-furfural dans l'étape (d) est le méthanol.

13. Procédé selon la revendication 11 ou 12, **caractérisé en ce que** la base dans l'étape (e) est le carbonate de potassium.

14. Procédé selon l'une quelconque des revendications 11 à 13, **caractérisé en ce que** la récupération du 5-hydroxyméthyl-furfural dans l'étape (f) comprend une distillation à trajet court du 5-hydroxyméthyl-furfural.

15. Procédé selon l'une quelconque des revendications 11 à 13, **caractérisé en ce que** la récupération du 5-hydroxyméthyl-furfural dans l'étape (f) comprend la cristallisation du 5-hydroxyméthyl-furfural dans un solvant approprié.
